(19) Europäisches Patentamt
European Patent Office
Office européen des brevets

(11) EP 4 729 504 A1

(12) **EUROPEAN PATENT APPLICATION**

(43) Date of publication:
**22.04.2026 Bulletin 2026/17**

(21) Application number: **25208247.4**

(22) Date of filing: **13.10.2025**

(51) International Patent Classification (IPC):
***C07C 41/48*** *(2006.01)* ***C07C 45/51*** *(2006.01)*
***C07F 3/02*** *(2006.01)*

(52) Cooperative Patent Classification (CPC):
(C-Sets available)
**C07C 41/48; C07C 45/515; C07F 3/02** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **17.10.2024 JP 2024181432**

(71) Applicant: **SHIN-ETSU CHEMICAL CO., LTD.**
**Tokyo 100-0005 (JP)**

(72) Inventors:
- **TSUKAGUCHI, Shogo**
  **Niigata, 942-8601 (JP)**
- **MIYAKE, Yuki**
  **Niigata, 942-8601 (JP)**
- **WATANABE, Takeru**
  **Niigata, 942-8601 (JP)**
- **NAGAE, Yusuke**
  **Niigata, 942-8601 (JP)**

(74) Representative: **De Vries & Metman**
**Overschiestraat 180**
**1062 XK Amsterdam (NL)**

# (54) (6Z)-6-TRIDECENYL METAL COMPOUND, PROCESS FOR PREPARING THE SAME, AND PROCESS FOR PREPARING (7Z)-7-TETRADECENAL

(57) The present invention provides a (6*Z*)-6-tridecenyl metal compound (1), wherein $M^1$ represents Li or $MgZ^1$, and $Z^1$ represents a halogen atom or a (6*Z*)-6-tridecenyl group. The present invention further provides the process for preparing the (6*Z*)-6-tridecenyl metal compound (1), comprising steps of preparing a (3*Z*)-3-decenyl metal compound (3), wherein $M^2$ represents Li or $MgZ^2$, and $Z^2$ represents a halogen atom or a (3*Z*)-3-decenyl group, from a 1-halo-(3*Z*)-3-decene compound (2), wherein $X^1$ represents a halogen atom; subjecting the (3*Z*)-3-decenyl metal compound (3) to a coupling reaction with a 1,3-dihalopropane compound (4), wherein $X^2$ and $X^3$ represent, independently of each other, a halogen atom, to form a 1-halo-(6*Z*)-6-tridecene compound (5), wherein $X^4$ represents a halogen atom; and preparing the aforesaid (6*Z*)-6-tridecenyl metal compound (1) from the 1-halo-(6*Z*)-6-tridecene compound (5).

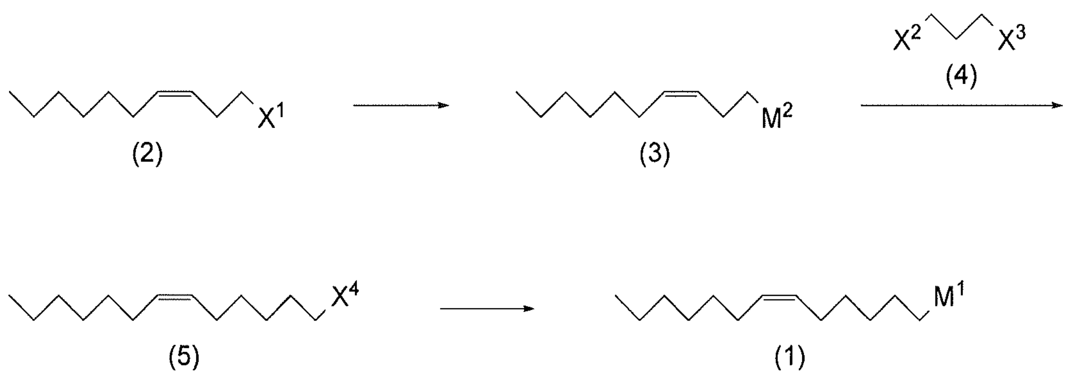

EP 4 729 504 A1

(52) Cooperative Patent Classification (CPC): (Cont.)

C-Sets
**C07C 41/48, C07C 43/303;**
**C07C 45/515, C07C 47/21**

## Description

### TECHNICAL FIELD

The present invention relates to a (6*Z*)-6-tridecenyl metal compound, a process for preparing a (6*Z*)-6-tridecenyl metal compound, and a process for preparing (7*Z*)-7-tetradecenal.

### BACKGROUND ART

The Olive moth (scientific name: *Prays oleae*) has been one of the most serious pests in olive-growing areas along the Mediterranean coast, owing to its larvae penetrating and feeding on the fruit of the olive. Traditional control methods of the Olive moth have included pesticides, but biological control methods are attracting attention due to concerns about residual pesticides and environmental impact, and one promising method is the use of sex pheromones (Non-Patent Literature 1 below).

The sex pheromone of the Olive moth is reported to be (7*Z*)-7-tetradecenal (Non-Patent Literature 2 below).

The following process is a known example of a process for preparing the aforesaid (7*Z*)-7-tetradecenal. As reported in Non-Patent Literature 3 below, n-butyllithium is added to 1-octyne at -45°C in tetrahydrofuran, then 6-bromohexyl 2-tetrahydropyranyl ether and hexamethylphosphoric triamide (HMPA) are added at -45°C, and the reaction mixture is subjected to a nucleophilic substitution reaction to form 7-tetradecyn-1-yl 2-tetrahydropyranyl ether. The tetrahydropyranyl group of the 7-tetradecyn-1-yl 2-tetrahydropyranyl ether thus obtained is then eliminated in methanol in the presence of 10-camphorsulfonic acid (CSA) to form 7-tetradecyn-1-ol. The 7-tetradecyn-1-ol is then subjected to a hydrogenation reaction in hexane in the presence of a Lindlar catalyst to form (7*Z*)-7-tetradecen-1-ol. The (7*Z*)-7-tetradecen-1-ol thus obtained is then subjected to the Swern oxidation with oxalyl chloride, dimethyl sulfoxide (DMSO), and triethylamine at -78°C in dichloromethane to form (7*Z*)-7-tetradecenal.

### PRIOR ART

[Non-Patent Literatures]

[Non-Patent Literature 1] E. M. Hegazi et al., 2009, Crop Protection, 28:181-189.
[Non-Patent Literature 2] D. G. Campion et al., 1979, Experientia, 35:1146-1147.
[Non-Patent Literature 3] Masao Shiozaki et al., 2003, Bull. Chem. Soc. Jpn., 76:1011-1022.


### OBJECT OF THE INVENTION

However, the preparation process reported in Non-Patent Literature 3 is not industrially practical due to the use of the solvent, hexamethylphosphoric triamide, which is carcinogenic and extremely toxic to the human body, and n-butyllithium, which is ignitable. Furthermore, the use of a Lindlar catalyst, which is an expensive catalyst of the noble metal, palladium, is not economical. Oxidation reactions often are accompanied by a danger of explosion. Moreover, the Swern oxidation reaction by-produces the toxic gas, carbon monoxide, and malodorous dimethyl sulfide and must be reacted at a low temperature near -78°C, which requires a special reactor and makes implementation on an industrial scale difficult.

The present invention has been made in view of the aforementioned circumstances, and aims to provide a novel synthetic intermediate useful for preparing (7*Z*)-7-tetradecenal, and a process for preparing the aforesaid synthetic intermediate. The present invention also aims to provide an efficient process for preparing (7*Z*)-7-tetradecenal from the aforesaid synthetic intermediate. The present invention also aims to provide an efficient and economical process for preparing (7*Z*)-7-tetradecenal from the aforesaid synthetic intermediate, and particularly a preparation process suited to industrially practical preparation.

### SUMMARY OF THE INVENTION

As a result of intensive research to overcome the aforesaid problems of the prior art, the present inventors found a novel compound, (6*Z*)-6-tridecenyl metal compound, as an intermediate useful for preparing (7*Z*)-7-tetradecenal. The present inventors also found a process for preparing the aforesaid novel compound, (6*Z*)-6-tridecenyl metal compound, and a process for preparing the sex pheromone of the Olive moth, (7*Z*)-7-tetradecenal, from the (6*Z*)-6-tridecenyl metal compound, and particularly a process for industrially preparing (7*Z*)-7-tetradecenal with fewer steps, and thus have completed the present invention. It was also found that the aforesaid process for preparing the aforesaid (6*Z*)-6-tridecenyl

metal compound and the aforesaid process for preparing the aforesaid (7*Z*)-7-tetradecenal are suited to industrial preparation of these compounds.

According to a first aspect of the present invention, there is provided a (6*Z*)-6-tridecenyl metal compound of the following general formula (1):

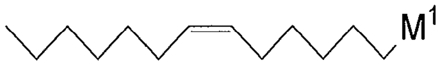 (1)

wherein $M^1$ represents Li or $MgZ^1$, and $Z^1$ represents a halogen atom or a (6*Z*)-6-tridecenyl group.

According to a second aspect of the present invention, there is provided a process for preparing a (6*Z*)-6-tridecenyl metal compound of the following general formula (1):

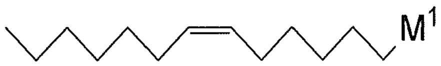 (1)

wherein $M^1$ represents Li or $MgZ^1$, and $Z^1$ represents a halogen atom or a (6*Z*)-6-tridecenyl group,
the process comprising steps of:
preparing a (3*Z*)-3-decenyl metal compound of the following general formula (3):

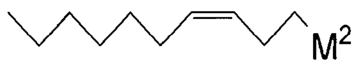 (3)

wherein $M^2$ represents Li or $MgZ^2$, and $Z^2$ represents a halogen atom or a (3*Z*)-3-decenyl group,
from a 1-halo-(3*Z*)-3-decene compound of the following general formula (2):

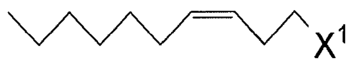 (2)

wherein $X^1$ represents a halogen atom;
subjecting the (3*Z*)-3-decenyl metal compound (3) to a coupling reaction with a 1,3-dihalopropane compound of the following general formula (4):

$X^2(CH_2)_3X^3$ (4)

wherein $X^2$ and $X^3$ represent, independently of each other, a halogen atom, to form a 1-halo-(6*Z*)-6-tridecene compound of the following general formula (5):

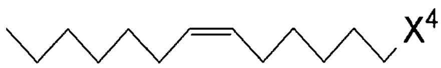 (5)

wherein $X^4$ represents a halogen atom; and
preparing the aforesaid (6*Z*)-6-tridecenyl metal compound (1) from the 1-halo-(6*Z*)-6-tridecene compound (5).

According to a third aspect of the present invention, there is provided a process for preparing (7*Z*)-7-tetradecenal of the following formula (8):

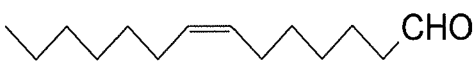 (8)

the process comprising steps of:
subjecting a (6*Z*)-6-tridecenyl metal compound of the following general formula (1):

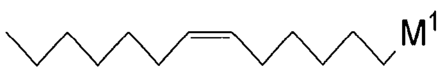 (1)

wherein $M^1$ represents Li or $MgZ^1$, and $Z^1$ represents a halogen atom or a (6*Z*)-6-tridecenyl group,
to a nucleophilic substitution reaction with an orthoformate ester compound of the following general formula (6):

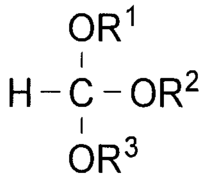

(6)

wherein $R^1$, $R^2$, and $R^3$ represent, independently of each other, an alkyl group having 1 to 6 carbon atoms, to form a 1,1-dialkoxy-(7*Z*)-7-tetradecene compound of the following general formula (7):

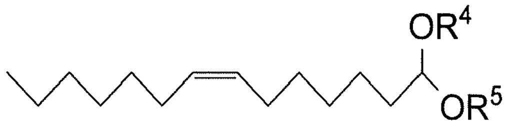

(7)

wherein $R^4$ and $R^5$ represent, independently of each other, an alkyl group having 1 to 6 carbon atoms; and subjecting the 1,1-dialkoxy-(7*Z*)-7-tetradecene compound (7) to a hydrolysis reaction to form the aforesaid (7*Z*)-7-tetradecenal (8).

According to a fourth aspect of the present invention, there is provided a process for preparing (7*Z*)-7-tetradecenal of the following formula (8):

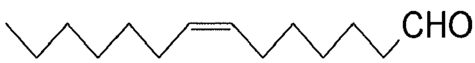

(8)

the process comprising a step of:
subjecting a (6*Z*)-6-tridecenyl metal compound of the following general formula (1):

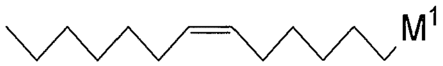

(1)

wherein $M^1$ represents Li or $MgZ^1$, and $Z^1$ represents a halogen atom or a (6*Z*)-6-tridecenyl group, to a nucleophilic substitution reaction with an *N,N*-dialkylformamide compound of the following general formula (9):

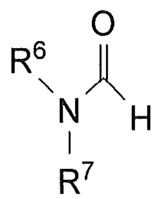

(9)

wherein $R^6$ and $R^7$ represent, independently of each other, an alkyl group having 1 to 3 carbon atoms, or $R^6$and $R^7$ may be bonded to each other to form a divalent alkylene group having 2 to 6 carbon atoms or a 3-oxa-1,5-pentanediyl group,
to form the aforesaid (7*Z*)-7-tetradecenal (8).

According to a fifth aspect of the present invention, there is provided the process for preparing (7*Z*)-7-tetradecenal (8) according to the aforesaid fourth aspect, wherein
the (6*Z*)-6-tridecenyl metal compound (1) is added dropwise to the *N,N*-dialkylformamide compound (9) in the aforesaid nucleophilic substitution reaction according to the aforesaid fourth aspect.

According to the present invention, the synthetic intermediate, (6*Z*)-6-tridecenyl metal compound, can be prepared, which is used for preparing (7*Z*)-7-tetradecenal with high yield but without using expensive starting material. The (6*Z*)-6-tridecenyl metal compound may be prepared as a key intermediate without using expensive starting materials, with high yield, and with fewer steps.

DETAILED DESCRIPTION OF THE INVENTION

A. (6Z)-6-Tridecenyl metal compound of the following general formula (1)

First, a (6Z)-6-tridecenyl metal compound of the following general formula (1) will be explained in detail below.

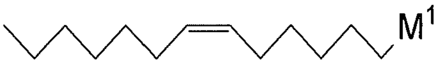

(1)

In the (6*Z*)-6-tridecenyl metal compound (1), $M^1$ represents Li or $MgZ^1$, and $Z^1$ represents a halogen atom or a (6*Z*)-6-tridecenyl group. Examples of the halogen atom $Z^1$ include a chlorine atom, a bromine atom, and an iodine atom.

Specific examples of the (6*Z*)-6-tridecenyl metal compound (1) include (6*Z*)-6-tridecenyllithium; and (6*Z*)-6-tridecenylmagnesium halide compounds (Grignard reagents) such as (6*Z*)-6-tridecenylmagnesium chloride, (6*Z*)-6-tridecenylmagnesium bromide, and (6*Z*)-6-tridecenylmagnesium iodide. (6*Z*)-6-Tridecenylmagnesium halide compounds are preferred. By using said (6*Z*)-6-tridecenylmagnesium halide compounds, a preferred availability may be ensured. (6*Z*)-6-Tridecenylmagnesium chloride is especially preferred. By using said (6*Z*)-6-tridecenylmagnesium chloride, an especially preferred availability may be ensured.

B. Process for preparing the (6*Z*)-6-tridecenyl metal compound (1)

One target compound of the present invention, (6*Z*)-6-tridecenyl metal compound (1), is prepared from a 1-halo-(3*Z*)-3-decene compound of the following general formula (2) according to, for example, a preparation process of the following chemical reaction formula:

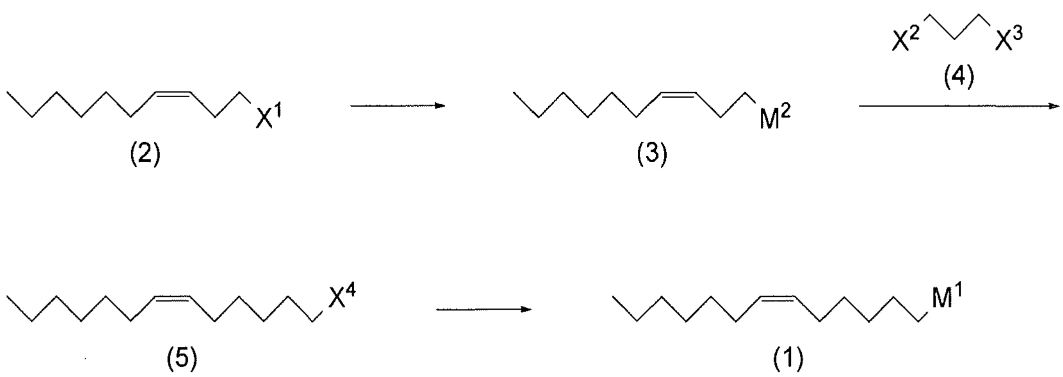

(4)

(2)

(3)

(5)

(1)

Specifically, a (6*Z*)-6-tridecenyl metal compound (1) is formed by the following steps of (i) preparing a (3*Z*)-3-decenyl metal compound (3) from a 1-halo-(3*Z*)-3-decene compound (2); (ii) subjecting the (3*Z*)-3-decenyl metal compound (3) to a coupling reaction with a 1,3-dihalopropane compound (4) to form a 1-halo-(6*Z*)-6-tridecene compound (5); and (iii) preparing the aforesaid (6*Z*)-6-tridecenyl metal compound (1) from the 1-halo-(6*Z*)-6-tridecene compound (5).

(i) Step of preparing a (3*Z*)-3-decenyl metal compound (3) from the 1-halo-(3*Z*)-3-decene compound (2)

(a) The 1-halo-(3*Z*)-3-decene compound (2) will be explained in detail below.

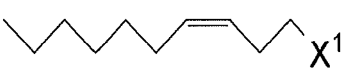

(2)

In the 1-halo-(3*Z*)-3-decene compound (2), $X^1$ represents a halogen atom. Examples of the halogen atom $X^1$ include a chlorine atom, a bromine atom, and an iodine atom.

Specific examples of the 1-halo-(3*Z*)-3-decene compound (2) include 1-chloro-(3*Z*)-3-decene, 1-bromo-(3*Z*)-3-decene, and 1-iodo-(3*Z*)-3-decene.

The 1-halo-(3*Z*)-3-decene compound (2) may be used alone or in combination thereof, if necessary. The 1-halo-(3*Z*)-3-decene compound (2) may be a commercially available one or may be synthesized in house. The 1-halo-(3*Z*)-3-decene compound (2) may be synthesized, for example, by reducing the carbon-carbon triple bond of 3-decyn-1-ol to form (3*Z*)-3-decen-1-ol, and then by halogenating the hydroxy group.

(b) The (3*Z*)-3-decenyl metal compound (3) will be explained in detail below.

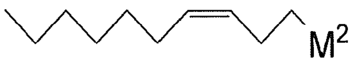

(3)

In the (3*Z*)-3-decenyl metal compound (3), $M^2$ represents Li or $MgZ^2$, and $Z^2$ represents a halogen atom or a (3*Z*)-3-decenyl group. Examples of the halogen atom include a chlorine atom, a bromine atom, and an iodine atom.

Specific examples of the (3*Z*)-3-decenyl metal compound (3) include (3*Z*)-3-decenyllithium; and (3*Z*)-3-decenylmagnesium halide compounds (Grignard reagents) such as (3*Z*)-3-decenylmagnesium chloride, (3*Z*)-3-decenylmagnesium bromide, and (3*Z*)-3-decenylmagnesium iodide. (3*Z*)-3-Decenylmagnesium halide compounds are preferred. By using said (3*Z*)-3-decenylmagnesium halide compounds, a preferred availability may be ensured. (3*Z*)-3-Decenylmagnesium chloride is especially preferred. By using said (3*Z*)-3-decenylmagnesium chloride, an especially preferred availability may be ensured.

(c) The step of preparing the (3*Z*)-3-decenyl metal compound (3) from the 1-halo-(3*Z*)-3-decene compound (2) will be explained in detail below.

The (3*Z*)-3-decenyl metal compound (3) may be prepared by a step of preparing the (3*Z*)-3-decenyl metal compound (3) from the 1-halo-(3*Z*)-3-decene compound of the general formula (2) according to the following chemical reaction formula.

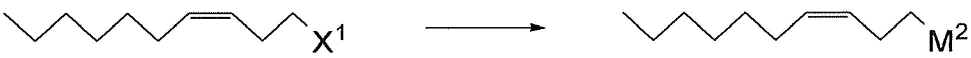

(2) (3)

(c-1) (3*Z*)-3-Decenyl metal compound (3) when $M^2 = MgZ^2$, i.e., (3*Z*)-3-decenylmagnesium halide compound (3: $M^2 = MgZ^2$) (a Grignard reagent)

The (3*Z*)-3-decenylmagnesium halide compound (3: $M^2 = MgZ^2$) may be prepared by a step of reacting a 1-halo-(3*Z*)-3-decene compound (2) with magnesium to form the (3*Z*)-3-decenylmagnesium halide compound (3: $M^2 = MgZ^2$) as shown by the following chemical reaction formula.

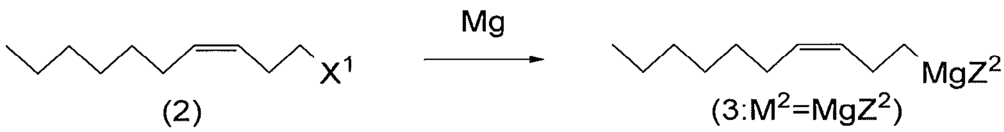


(2) (3:$M^2$=$MgZ^2$)

When preparing the aforesaid Grignard reagent, the amount of magnesium used, per mol of the 1-halo-(3*Z*)-3-decene compound (2), is preferably 1.0 to 2.0 gram atoms. By using said amount, a preferred completion of the reaction may be ensured.

Examples of a solvent used to prepare the aforesaid Grignard reagent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. Ether solvents such as tetrahydrofuran, 2-methyltetrahydrofuran, diethyl ether, and 4-methyltetrahydropyran are preferred. By using said ether solvents such as tetrahydrofuran, 2-methyltetrahydrofuran, diethyl ether, and 4-methyltetrahydropyran, a preferred reaction rate of forming the aforesaid Grignard reagent may be ensured. Tetrahydrofuran and 2-methyltetrahydrofuran are more preferred. By using said tetrahydrofuran and 2-methyltetrahydrofuran, a more preferred reaction rate of forming the aforesaid Grignard reagent may be ensured.

The solvent may be used alone or in combination thereof, if necessary. The solvent may be a commercially available one.

The amount of the solvent used, per mol of the 1-halo-(3*Z*)-3-decene compound (2), is preferably 30 to 5,000 g, more preferably 50 to 3,000 g, even more preferably 70 to 1,500 g, and especially preferably 150 to 800 g. By using said preferred amount, said more preferred amount, said even more preferred amount, and said especially preferred amount, a preferred reactivity, a more preferred reactivity, an even more preferred reactivity, and an especially preferred reactivity may be ensured.

The reaction temperature of the preparation of the aforesaid Grignard reagent varies, depending on the solvent to be used, and is preferably 30 to 120°C. By using said reaction temperature, a preferred reactivity may be ensured.

The reaction time of the preparation of the aforesaid Grignard reagent varies, depending on the solvent to be used and/or the production scale, and is preferably 0.5 to 100 hours. By using said reaction time, a preferred reactivity may be ensured.

(c-2) (3*Z*)-3-Decenyl metal compound (3) when $M^2$ = Li, i.e., (3*Z*)-3-decenyllithium (3: $M^2$ = Li)

(3*Z*)-3-Decenyllithium (3: $M^2$ = Li) may be prepared by a step of reacting a 1-halo-(3*Z*)-3-decene compound (2) with lithium to form (3*Z*)-3-decenyllithium (3: $M^2$ = Li) as shown by the following chemical reaction formula.

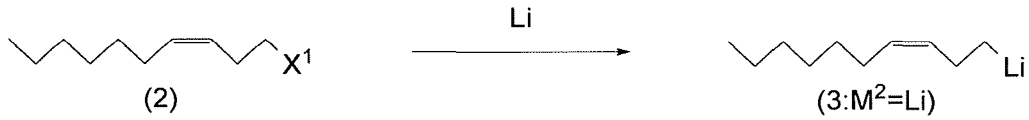

When preparing (3*Z*)-3-decenyllithium (3: $M^2$ = Li) from the 1-halo-(3*Z*)-3-decene compound (2), the amount of lithium used, per mol of the 1-halo-(3*Z*)-3-decene compound (2), is preferably 2.0 to 4.0 gram atoms. By using said amount, a preferred completion of the reaction may be ensured.

Examples of a solvent used in the preparation of (3*Z*)-3-decenyllithium (3: $M^2$ = Li) from the 1-halo-(3*Z*)-3-decene compound (2) include ether solvents such as tetrahydrofuran and diethyl ether; and hydrocarbon solvents such as pentane, hexane, cyclohexane, heptane, benzene, toluene, xylene, and cumene. Diethyl ether, pentane, hexane, cyclohexane, and heptane are preferred. By using said diethyl ether, pentane, hexane, cyclohexane, and heptane, a preferred reactivity or stability of (3*Z*)-3-decenyllithium (3: $M^2$ = Li) may be ensured.

The solvent may be used alone or in combination thereof, if necessary. The solvent may be a commercially available one.

The amount of the solvent used, per mol of the 1-halo-(3*Z*)-3-decene compound (2), is preferably 50 to 4,000 g, more preferably 70 to 2,000 g, even more preferably 100 to 1,000 g, and especially preferably 150 to 600 g. By using said preferred amount, said more preferred amount, said even more preferred amount, and said especially preferred amount, a preferred reactivity, a more preferred reactivity, an even more preferred reactivity, and an especially preferred reactivity may be ensured.

The reaction temperature of the preparation of (3*Z*)-3-decenyllithium (3: $M^2$ = Li) from the 1-halo-(3*Z*)-3-decene compound (2) varies, depending on the solvent to be used, and is preferably -30°C to 60°C. By using said reaction temperature, a preferred reactivity may be ensured.

The reaction time of the preparation of (3*Z*)-3-decenyllithium (3: $M^2$ = Li) from the 1-halo-(3*Z*)-3-decene compound (2) varies, depending on the solvent to be used and/or the production scale, and is preferably 0.5 to 100 hours. By using said reaction time, a preferred reactivity may be ensured.

(ii) Step of subjecting the (3*Z*)-3-decenyl metal compound (3) to coupling reaction with the 1,3-dihalopropane compound (4) to form the 1-halo-(6*Z*)-6-tridecene compound (5)

(a) The 1,3-dihalopropane compound (4) will be explained in detail below.

$$X^2(CH_2)_3X^3 \qquad (4)$$

In the general formula (4) above, $X^2$ and $X^3$ represent, independently of each other, a halogen atom. Examples of the halogen atoms $X^2$ and $X^3$ include a chlorine atom, a bromine atom, and an iodine atom.

Examples of combinations of $X^2$ and $X^3$ include a chlorine atom with a chlorine atom, a bromine atom with a chlorine atom, a chlorine atom with an iodine atom, a bromine atom with a bromine atom, a bromine atom with an iodine atom, and an iodine atom with an iodine atom.

Specific examples of the 1,3-dihalopropane compound (4) include 1,3-dichloropropane, 1,3-dibromopropane, 1,3-diiodopropane, 1-bromo-3-chloropropane, 1-chloro-3-iodopropane, and 1-bromo-3-iodopropane.

1-Bromo-3-chloropropane, 1-chloro-3-iodopropane, and 1-bromo-3-iodopropane are especially preferred. By using said 1-bromo-3-chloropropane, 1-chloro-3-iodopropane, and 1-bromo-3-iodopropane, an especially preferred yield may be ensured.

The 1,3-dihalopropane compound (4) may be used alone or in combination thereof, if necessary. The 1,3-dihalopropane compound (4) may be a commercially available one or may be synthesized in house.

The 1,3-dihalopropane compound (4) may be synthesized, for example, by halogenating 1,3-propanediol.

(b) A coupling reaction of the (3*Z*)-3-decenyl metal compound (3) with the 1,3-dihalopropane compound (4) will be explained in detail below.

In the coupling reaction, the amount of the (3Z)-3-decenyl metal compound (3) used, per mol of the 1,3-dihalopropane compound (4), is preferably 0.4 to 1.4 mol. By using said amount, preferred economy may be ensured.

A solvent may be incorporated in the coupling reaction, if necessary. Examples of the solvent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N*-dimethylformamide (DMF), *N,N*-dimethylacetamide (DMAC), *N*-methylpyrrolidone (NMP), dimethyl sulfoxide (DMSO), γ-butyrolactone (GBL), acetonitrile, acetone,

*N,N'*-dimethylpropylene urea (DMPU), hexamethylphosphoric triamide (HMPA), dichloromethane, and chloroform. Tetrahydrofuran, 2-methyltetrahydrofuran, 4-methyltetrahydropyran, toluene, and acetonitrile are preferred. By using said tetrahydrofuran, 2-methyltetrahydrofuran, 4-methyltetrahydropyran, toluene, and acetonitrile, a preferred reactivity may be ensured. Tetrahydrofuran, 2-methyltetrahydrofuran, and 4-methyltetrahydropyran are more preferred. By using said tetrahydrofuran, 2-methyltetrahydrofuran, and 4-methyltetrahydropyran, a more preferred reactivity may be ensured.

The solvent may be used alone or in combination thereof, if necessary. The solvent may be a commercially available one.

The amount of the solvent used, per mol of the 1,3-dihalopropane compound (4), is preferably 30 to 5,000 g, more preferably 50 to 3,000 g, and especially preferably 70 to 1,000 g. By using said preferred amount, said more preferred amount, and said especially preferred amount, a preferred reactivity, a more preferred reactivity, and an especially preferred reactivity may be ensured.

The coupling reaction may be carried out in the presence of a catalyst, if necessary. Examples of the catalyst include copper compounds such as cuprous halides such as cuprous chloride, cuprous bromide, and cuprous iodide, and cupric halides such as cupric chloride, cupric bromide, and cupric iodide; iron compounds such as iron(II) chloride, iron(III) chloride, iron(II) bromide, iron(III) bromide, iron(II) iodide, iron(III) iodide, and iron(III) acetylacetonate; silver compounds such as silver chloride, silver nitrate, and silver acetate; titanium compounds such as titanium tetrachloride, titanium tetrabromide, titanium(IV) methoxide, titanium(IV) ethoxide, titanium(IV) isopropoxide, and titanium(IV) oxide; palladium(II) compounds such as dichlorobis(triphenylphosphine)palladium and dichloro[1,1'-bis(diphenylphosphino)ferrocene] palladium; and nickel compounds such as nickel chloride, dichloro[1,2-bis(diphenylphosphino)ethane]nickel(II), and dichlorobis(triphenylphosphine)nickel(II). Copper compounds are preferred. By using said copper compounds, a preferred reactivity and/or economy may be ensured. Cuprous halides such as cuprous chloride, cuprous bromide, and cuprous iodide are more preferred. By using said cuprous halides such as cuprous chloride, cuprous bromide, and cuprous iodide, a more preferred reactivity and/or economy may be ensured. Cuprous iodide is especially preferred. By using said cuprous iodide, an especially preferred reactivity and/or economy may be ensured.

The catalyst may be used alone or in combination thereof, if necessary. The catalyst may be a commercially available one.

The amount of the catalyst used, per mol of the 1,3-dihalopropane compound (4), is preferably 0.0001 to 1.00 mol, and more preferably 0.001 to 0.300 mol. By using said preferred amount, a preferred reaction rate and/or post-treatment may be ensured. By using said more preferred amount, a more preferred reaction rate and/or post-treatment may be ensured.

When the coupling reaction is carried out in the presence of a catalyst, a co-catalyst may be used, if necessary. Examples of the co-catalyst include trialkyl phosphite compounds having 3 to 9 carbon atoms such as triethyl phosphite; and arylphosphine compounds having 18 to 44 carbon atoms such as triphenylphosphine, tritolylphosphine, and 2,2'-bis(diphenylphosphino)-1,1'-binaphthyl (BINAP). Trialkyl phosphite is preferred. By using said trialkyl phosphite, a preferred reactivity may be ensured. Triethyl phosphite is especially preferred. By using said triethyl phosphite, an especially preferred reactivity may be ensured.

The co-catalyst may be used alone or in combination thereof, if necessary. The co-catalyst may be a commercially available one.

The amount of the co-catalyst used, per mol of the 1,3-dihalopropane compound (4), is preferably 0.0001 to 1.00 mol, and more preferably 0.001 to 0.300 mol. By using said preferred amount and said more preferred amount, a preferred reactivity and a more preferred reactivity may be ensured.

When the coupling reaction is carried out in the presence of a catalyst, a lithium salt may be added, if necessary. Examples of the lithium salt include lithium halides such as lithium chloride, lithium bromide, and lithium iodide, lithium nitrate, and lithium carbonate. Lithium nitrate and lithium halides such as lithium chloride are preferred. By using said lithium nitrate and lithium halides such as lithium chloride, a preferred reactivity may be ensured.

The lithium salt may be used alone or in combination thereof, if necessary. The lithium salt may be a commercially available one.

The amount of the lithium salt used in the coupling reaction, per mol of the 1,3-dihalopropane compound (4), is preferably 0.005 to 0.250 mol. By using said preferred amount, a preferred reactivity may be ensured.

The reaction temperature of the coupling reaction varies, depending on the (3*Z*)-3-decenyl metal compound (3) used, and is preferably -78 to 70°C, and more preferably -20 to 35°C. By using said preferred reaction temperature and said more preferred reaction temperature, a preferred reactivity and/or quality and a more preferred reactivity and/or quality may be ensured. The reaction temperature is even more preferably -15 to 30°C, and especially preferably -5 to 25°C. By using said even more preferred reaction temperature, an even more preferred reactivity and/or quality (i.e., geometrical isomerism), particularly an even more preferred quality (i.e., geometrical isomerism), may be ensured. By using said especially preferred reaction temperature, an especially preferred reactivity and/or quality (i.e., geometrical isomerism), particularly an especially preferred quality (i.e., geometrical isomerism), may be ensured.

The inventors found, as a result of intensive research, that when the coupling reaction was carried out at a

reaction temperature of 35 to 40°C, compared to a temperature of less than 30°C such as, for example, -5 to 25°C, geometrical isomerization of the carbon-carbon double bond drastically proceeded and the yield was reduced by nearly 10%, which was contrary to expectations. Analysis showed that geometrical isomerization of the carbon-carbon double bond substantially did not proceed in the course of forming the (3Z)-3-decenylmagnesium halide compound (3) from the 1-halo-(3Z)-3-decene compound (2), and so it may be inferred that the geometrical isomerization of the carbon-carbon double bond proceeded in the coupling reaction step. It is noted that, as shown in Reference Example 1 below, such geometrical isomerization of the carbon-carbon double bond did not proceed in a coupling reaction of a similar Grignard reagent with a similar dihaloalkane.

The reaction time of the coupling reaction varies, depending on the solvent to be used and/or the production scale, and is preferably 0.5 to 100 hours. By using said preferred reaction time, a preferred reactivity may be ensured.

When $X^2$ and $X^3$ are different from each other in the general formula (4) above, the halogen atom that is more reactive may be preferentially reacted in the coupling reaction by appropriately selecting the aforesaid catalyst, the aforesaid co-catalyst, the aforesaid reaction temperature, or a combination of these factors. The 1-halo-(6*Z*)-6-tridecene compound (5) may be formed so that $X^4$ is, for example, a chlorine atom by using a 1,3-dihalopropane compound (4) in which the combination of mutually-different $X^2$ and $X^3$ is a chlorine atom with a bromine atom or a chlorine atom with an iodine atom. The 1-halo-(6*Z*)-6-tridecene compound (5) may be formed so that $X^4$ is a bromine atom by using a 1,3-dihalopropane compound (4) in which the combination of mutually-different $X^2$ and $X^3$ is a bromine atom with an iodine atom.

(c) The 1-halo-(6*Z*)-6-tridecene compound (5) will be explained in detail below.

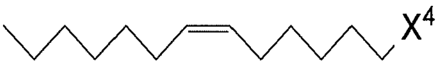

(5)

In the general formula (5) above, $X^4$ represents a halogen atom.

Specific examples of the halogen atom $X^4$ include a chlorine atom, a bromine atom, and an iodine atom. A chlorine atom and a bromine atom are preferred. By using said chlorine atom and bromine atom, a preferred stability may be ensured. A chlorine atom is especially preferred. By using said chlorine atom, an especially preferred stability may be ensured.

Specific examples of the 1-halo-(6*Z*)-6-tridecene compound (5) include 1-chloro-(6*Z*)-6-tridecene, 1-bromo-(6*Z*)-6-tridecene, and 1-iodo-(6*Z*)-6-tridecene.

(iii) Step of preparing (6*Z*)-6-tridecenyl metal compound (1) from 1-halo-(6*Z*)-6-tridecene compound (5)

A (6*Z*)-6-tridecenyl metal compound (1) may be prepared by a step of forming the (6*Z*)-6-tridecenyl metal compound (1) from the aforesaid 1-halo-(6*Z*)-6-tridecene compound of the general formula (5) of the following chemical reaction formula.

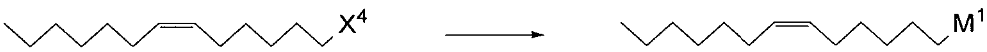

(5) (1)

A (6*Z*)-6-tridecenylmagnesium halide compound (1: $M^1$ = $MgZ^1$) (a Grignard reagent) may be prepared, for example, by reacting the aforesaid 1-halo-(6*Z*)-6-tridecene compound of the following general formula (5) with magnesium as shown by the following chemical reaction formula:

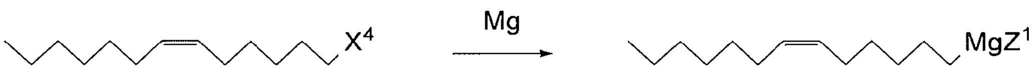


(5) (1:$M^1$=$MgZ^1$)

The 1-halo-(6Z)-6-tridecene compound (5) may be used alone or in combination thereof, if necessary.

The amount of magnesium used to prepare the aforesaid Grignard reagent, per mol of the 1-halo-(6Z)-6-tridecene compound (5), is preferably 1.0 to 2.0 gram atoms. By using said preferred amount, a preferred completion of the reaction may be ensured.

Examples of a solvent in the preparation of the aforesaid Grignard reagent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. Ether solvents such as tetrahydrofuran, 2-methyltetrahydrofuran, diethyl ether, and 4-methyltetrahydropyran are preferred. By using said ether solvents such as tetrahydrofuran, 2-methyltetrahydrofuran, diethyl ether, and 4-methyltetrahydropyran, a preferred reaction rate of Grignard reagent formation may be ensured. Tetrahydrofuran and 2-methyltetrahydrofuran are more preferred. By using said tetrahydrofuran and 2-methyltetrahydrofuran, a more preferred reaction rate of Grignard reagent formation may be ensured.

The solvent may be used alone or in combination thereof, if necessary. The solvent may be a commercially available one.

The amount of the solvent used, per mol of the 1-halo-(6*Z*)-6-tridecene compound (5), is preferably 30 to 5,000 g, more preferably 50 to 3,000 g, even more preferably 70 to 1,500 g, and especially preferably 150 to 800 g. By using said preferred amount, said more preferred amount, said even more preferred amount, and said especially preferred amount, a preferred reactivity, a more preferred reactivity, an even more preferred reactivity, and an especially preferred reactivity may be ensured.

The reaction temperature of the preparation of the Grignard reagent varies, depending on the solvent to be used, and is preferably 30 to 120°C. By using said preferred reaction temperature, a preferred reactivity may be ensured.

The reaction time of the preparation of the Grignard reagent varies, depending on the solvent to be used and/or the production scale, and is preferably 0.5 to 100 hours. By using said preferred reaction time, a preferred reactivity may be ensured.

(6*Z*)-6-Tridecenyllithium (1: $M^1$ = Li) may be prepared, for example, by a step of reacting a 1-halo-(6*Z*)-6-tridecene compound (5) with lithium to form (6*Z*)-6-tridecenyllithium (1: $M^1$ = Li) as shown by the following chemical reaction formula.

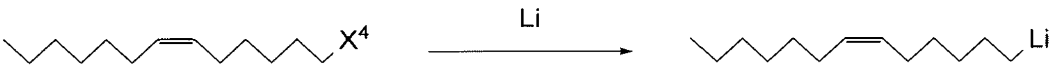


(5) (1:$M^1$=Li)

The 1-halo-(6*Z*)-6-tridecene compound (5) may be used alone or in combination thereof, if necessary.

The amount of lithium used to form (6*Z*)-6-tridecenyllithium (1: $M^1$ = Li) from the 1-halo-(6*Z*)-6-tridecene compound (5), per mol of the 1-halo-(6*Z*)-6-tridecene compound (5), is preferably 2.0 to 4.0 gram atoms. By using said preferred amount, a preferred completion of the reaction may be ensured.

Examples of a solvent used to form (6*Z*)-6-tridecenyllithium (1: $M^1$ = Li) from the 1-halo-(6*Z*)-6-tridecene compound (5) include ether solvents such as tetrahydrofuran and diethyl ether; and hydrocarbon solvents such as pentane, hexane, cyclohexane, heptane, benzene, toluene, xylene, and cumene. Diethyl ether, pentane, hexane, cyclohexane, and heptane are preferred. By using said diethyl ether, pentane, hexane, cyclohexane, and heptane, a preferred reactivity or stability of (6*Z*)-6-tridecenyllithium (1: $M^1$ = Li) may be ensured.

The amount of the solvent used, per mol of the 1-halo-(6*Z*)-6-tridecene compound (5), is preferably 50 to 4,000 g, more preferably 70 to 2,000 g, even more preferably 100 to 1,000 g, and especially preferably 150 to 600 g. By using said preferred amount, said more preferred amount, said even more preferred amount, and said especially preferred amount, a preferred reactivity, a more preferred reactivity, an even more preferred reactivity, and an especially preferred reactivity may be ensured.

The reaction temperature of the preparation of (6*Z*)-6-tridecenyllithium (1: $M^1$ = Li) from the 1-halo-(6*Z*)-6-tridecene compound (5) varies, depending on the solvent to be used, and is preferably -30 to 60°C. By using said preferred reaction temperature, a preferred reactivity may be ensured.

The reaction time of the preparation of (6*Z*)-6-tridecenyllithium (1: $M^1$ = Li) from the 1-halo-(6*Z*)-6-tridecene compound (5) varies, depending on the solvent to be used and/or the production scale, and is preferably 0.5 to 100 hours. By using said preferred reaction time, a preferred reactivity may be ensured.

Different processes for preparing (7*Z*)-7-tetradecenal (8) from the (6*Z*)-6-tridecenyl metal compound (1) will be explained in detail below in Sections C and D.

C. Process for preparing (7*Z*)-7-tetradecenal (8) from starting materials, (6*Z*)-6-tridecenyl metal compound (1) and orthoformate ester compound (6)

One target compound of the present invention, (7*Z*)-7-tetradecenal (8), may be prepared from a (6*Z*)-6-tridecenyl metal compound (1) and an orthoformate ester compound (6) according to the preparation process of the following chemical reaction formula:

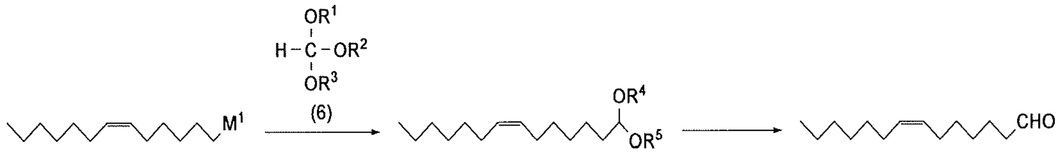


(1) (7) (8)

Specifically, (7*Z*)-7-tetradecenal (8) is formed by the following steps of (i) subjecting a (6*Z*)-6-tridecenyl metal compound (1) to a nucleophilic substitution reaction with an orthoformate ester compound (6) to form a 1,1-dialkoxy-(7*Z*)-7-tetradecene compound (7); and (ii) subjecting the 1,1-dialkoxy-(7*Z*)-7-tetradecene compound (7) to a hydrolysis reaction to form (7*Z*)-7-tetradecenal (8).

(i) Step of subjecting (6*Z*)-6-tridecenyl metal compound (1) to nucleophilic substitution reaction with the orthoformate ester compound (6) to form the 1,1-dialkoxy-(7*Z*)-7-tetradecene compound (7)

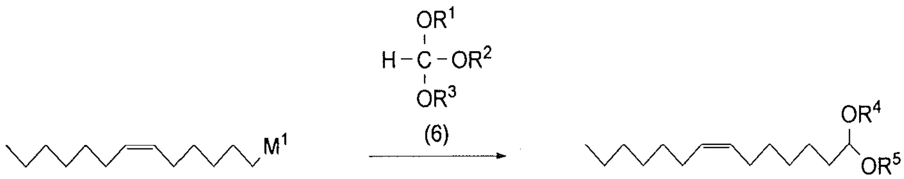


(1) (7)

(a) The orthoformate ester compound (6) will be explained in detail below.

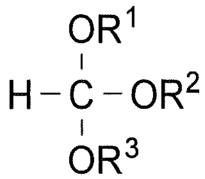
(6)

In the general formula (6) above, $R^1$, $R^2$, and $R^3$ represent, independently of each other, an alkyl group having 1 to 6 carbon atoms, and preferably 1 to 3 carbon atoms.

Examples of the alkyl groups having 1 to 6 carbon atoms include linear alkyl groups such as a methyl group, an ethyl group, an n-propyl group, an n-butyl group, an n-pentyl group, and an n-hexyl group; and branched alkyl groups such as an isopropyl group and an isobutyl group.

Specific examples of the orthoformate ester compound (6) include methyl orthoformate, ethyl orthoformate, propyl orthoformate, butyl orthoformate, pentyl orthoformate, and hexyl orthoformate. Methyl orthoformate and ethyl orthoformate are preferred. By using said methyl orthoformate and ethyl orthoformate, a preferred availability may be ensured.

The orthoformate ester compound (6) may be used alone or in combination thereof, if necessary. The orthoformate ester compound (6) may be a commercially available one.

(b) A nucleophilic substitution reaction of the (6*Z*)-6-tridecenyl metal compound (1) with the orthoformate ester compound (6) will be explained in detail below.

The amount of the (6*Z*)-6-tridecenyl metal compound (1) used, per mol of the orthoformate ester compound (6), is preferably 0.5 to 1.5 mol. By using said preferred amount, preferred economy may be ensured.

Examples of a solvent used in the nucleophilic substitution reaction include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. Tetrahydrofuran, 2-methyltetrahydrofuran, 4-methyltetrahydropyran, and toluene are preferred. By using said tetrahydrofuran, 2-methyltetrahydrofuran, 4-methyltetrahydropyran, and toluene, a preferred reactivity may be ensured.

The solvent may be used alone or in combination thereof, if necessary. The solvent may be a commercially available one.

When the (6*Z*)-6-tridecenyl metal compound (1) is diluted with a solvent or when a solvent is used to prepare the

(6*Z*)-6-tridecenyl metal compound (1), the same solvent or a different solvent may be incorporated in the nucleophilic substitution reaction.

When different solvents are used, a solvent that increases the reactivity may be substituted for the solvent of the nucleophilic substitution reaction.

When, for example, tetrahydrofuran is used as the solvent in the preparation of the (6*Z*)-6-tridecenyl metal compound (1) and toluene is selected as the solvent used in the nucleophilic substitution reaction, the solvent of the reaction system may be replaced with toluene by placing the (6*Z*)-6-tridecenyl metal compound (1) with tetrahydrofuran in a reactor, then adding the orthoformate ester compound (6) and toluene to the reactor, and subsequently distilling off tetrahydrofuran while increasing the reaction temperature.

The amount of the solvent used, per mol of the orthoformate ester compound (6), is preferably 100 to 6,000 g, and especially preferably 150 to 2,000 g. By using said preferred amount and said especially preferred amount, a preferred reactivity and an especially preferred reactivity may be ensured.

The reaction temperature of the nucleophilic substitution reaction is preferably 75 to 130°C, and especially preferably 80 to 120°C. By using said preferred reaction temperature and said especially preferred reaction temperature, a preferred smooth reaction with less solvent evaporation and an especially preferred smooth reaction with less solvent evaporation may be ensured.

The reaction time of the nucleophilic substitution reaction varies, depending on the solvent to be used or the production scale, and is preferably 0.5 to 100 hours.

(c) The 1,1-dialkoxy-(7*Z*)-7-tetradecene compound (7) will be explained in detail below.

In the 1,1-dialkoxy-(7*Z*)-7-tetradecene compound (7), $R^4$ and $R^5$ represent, independently of each other, an alkyl group having 1 to 6 carbon atoms, and preferably 1 to 3 carbon atoms.

Examples of the alkyl group having 1 to 6 carbon atoms include linear alkyl groups such as a methyl group, an ethyl group, an n-propyl group, an n-butyl group, an n-pentyl group, and an n-hexyl group; and branched alkyl groups such as an isopropyl group and an isobutyl group.

Specific examples of the 1,1-dialkoxy-(7*Z*)-7-tetradecene compound (7) include 1,1-dimethoxy-(7*Z*)-7-tetradecene, 1,1-diethoxy-(7*Z*)-7-tetradecene, 1,1-dipropyloxy-(7*Z*)-7-tetradecene, 1,1-dibutyloxy-(7*Z*)-7-tetradecene, 1,1-dipentyloxy-(7*Z*)-7-tetradecene, and 1,1-dihexyloxy-(7*Z*)-7-tetradecene.

(ii) Step of subjecting the 1,1-dialkoxy-(7*Z*)-7-tetradecene compound (7) to a hydrolysis reaction to form (7*Z*)-7-tetradecenal (8)

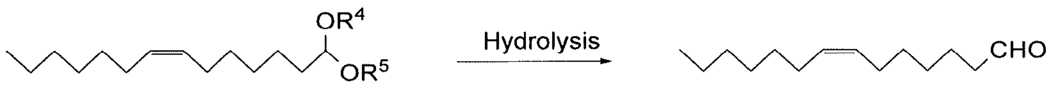


(7) (8)

In the aforesaid hydrolysis reaction, the 1,1-dialkoxy-(7Z)-7-tetradecene compound (7) may be used alone or in combination thereof, if necessary.

The hydrolysis reaction may be carried out, for example, with an acid and water.

Examples of the acid mentioned above include inorganic acids such as a hydrochloric acid and a hydrobromic acid; and *p*-toluenesulfonic acid, benzenesulfonic acid, trifluoroacetic acid, acetic acid, formic acid, oxalic acid, iodotrimethylsilane, and titanium tetrachloride. Acetic acid, formic acid, and oxalic acid are preferred. By using said acetic acid, formic acid, and oxalic acid, a preferred reactivity may be ensured. Acetic acid and formic acid are especially preferred. By using said acetic acid and formic acid, an especially preferred reactivity may be ensured.

The acid may be used alone or in combination thereof, if necessary. The acid may be a commercially available one.

The amount of the acid used, per mol of the 1,1-dialkoxy-(7*Z*)-7-tetradecene compound (7), is preferably 0.01 to 10.0 mol. By using said preferred amount, a preferred reactivity may be ensured.

The amount of the water used, per mol of the 1,1-dialkoxy-(7*Z*)-7-tetradecene compound (7), is preferably 18 to 7,000 g, more preferably 18 to 3,000 g, even more preferably 30 to 600 g, and especially preferably 40 to 200 g. By using said preferred amount, said more preferred amount, said even more preferred amount, and said especially preferred amount, a preferred reactivity, a more preferred reactivity, an even more preferred reactivity, and an especially preferred reactivity may be ensured.

In the hydrolysis reaction, a solvent may be further incorporated, if necessary, in addition to the acid or water.

Examples of the solvent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N,N*-dimethylformamide (DMF), *N,N*-dimethylacetamide (DMAC), *N*-methylpyrrolidone (NMP), dimethyl sulfoxide (DMSO), γ-butyrolactone (GBL), acetonitrile, acetone, *N,N'*-dimethylpropylene urea (DMPU), hexamethylphosphoric triamide (HMPA), dichloromethane, and chloroform; ester solvents such as methyl acetate, ethyl acetate, n-propyl acetate, and n-butyl acetate; and alcoholic solvents such as methanol and ethanol.

The solvent may be used alone or in combination thereof, if necessary. The solvent may be a commercially available one.

The optimal solvent varies, depending on the acid to be used. When, for example, oxalic acid is used as the acid, tetrahydrofuran, 2-methyltetrahydrofuran, acetone, and γ-butyrolactone are preferred. By using said tetrahydrofuran, 2-methyltetrahydrofuran, acetone, and γ-butyrolactone, a preferred reactivity may be ensured. When acetic acid or formic acid is used as the acid, tetrahydrofuran, 2-methyltetrahydrofuran, 1,4-dioxane, and toluene are preferred. By using said tetrahydrofuran, 2-methyltetrahydrofuran, 1,4-dioxane, and toluene, a preferred reactivity may be ensured.

The amount of the solvent used, per mol of the 1,1-dialkoxy-(7*Z*)-7-tetradecene compound (7), is preferably 0 to 7,000 g, more preferably 0 to 3,000 g, even more preferably 0 to 1,000 g, and especially preferably 0 to 150 g. By using said preferred amount, said more preferred amount, said even more preferred amount, and said especially preferred amount, a preferred reactivity, a more preferred reactivity, an even more preferred reactivity, and an especially preferred reactivity may be ensured.

The reaction temperature of the hydrolysis reaction varies, depending on the acid and/or the solvent to be used, and is preferably -15 to 180°C, and more preferably 40 to 120°C. By using said preferred reaction temperature and said more preferred reaction temperature, a preferred reactivity and a more preferred reactivity may be ensured.

The reaction time of the hydrolysis reaction varies, depending on the acid and the solvent to be used and/or the production scale, and is preferably 0.5 to 100 hours.

D. Process for preparing (7*Z*)-7-tetradecenal (8) from starting materials, (6*Z*)-6-tridecenyl metal compound (1) and *N,N*-dialkylformamide compound (9)

One target compound of the present invention, (7*Z*)-7-tetradecenal (8), may be prepared from a (6*Z*)-6-tridecenyl metal compound (1) and an *N,N*-dialkylformamide compound (9) according to a preparation process of the following chemical reaction formula:

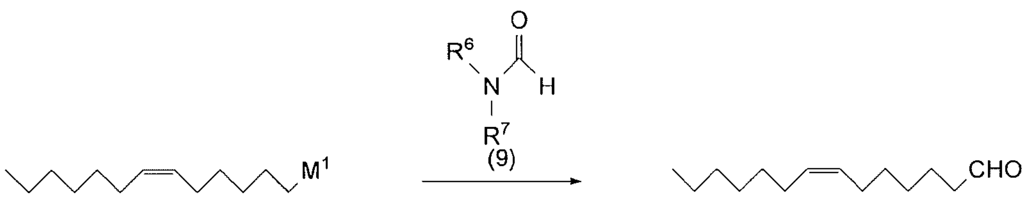

(1) (8)

Specifically, (7*Z*)-7-tetradecenal (8) is obtained by (i) a step of subjecting a (6*Z*)-6-tridecenyl metal compound (1) to a nucleophilic substitution reaction with an *N,N*-dialkylformamide compound (9) to form (7*Z*)-7-tetradecenal (8).

(i) Step of subjecting (6*Z*)-6-tridecenyl metal compound (1) to nucleophilic substitution reaction with *N,N*-dialkylformamide compound (9) to form (7*Z*)-7-tetradecenal (8)

(a) The *N,N*-dialkylformamide compound (9) will be explained in detail below.

In the *N,N*-dialkylformamide compound (9), $R^6$ and $R^7$ represent, independently of each other, an alkyl group having 1 to 3 carbon atoms, or $R^6$ and $R^7$ may be bonded to each other to form a divalent alkylene group having 2 to 6 carbon atoms, preferably 4 to 6 carbon atoms, and even more preferably 4 to 5 carbon atoms, or a 3-oxa-1,5-pentanediyl group. By using said divalent alkylene group having 4 to 6 carbon atoms and said divalent alkylene group having 4 to 5 carbon atoms, a preferred availability and an even more preferred availability may be ensured.

Examples of the alkyl group having 1 to 3 carbon atoms include linear alkyl groups such as a methyl group, an ethyl group, and an n-propyl group; and branched alkyl groups such as an isopropyl group. Examples of the divalent alkylene group having 2 to 6 carbon atoms include an ethylene group, a 1,3-propylene group, a 1,4-butylene group, a 1,5-pentylene group, and a 1,6-hexylene group.

Examples of the *N,N*-dialkylformamide compound (9) include *N,N*-dimethylformamide, *N*-ethyl-*N*-methylformamide, *N,N*-diethylformamide, *N,N*-diisopropylformamide, 1-formylpyrrolidine, 1-formylpiperidine, and 4-formylmorpholine. *N,N*-Dimethylformamide, *N,N*-diethylformamide, 1-formylpiperidine, and 4-formylmorpholine are preferred. By using said *N,N*-dimethylformamide, *N,N*-diethylformamide, 1-formylpiperidine, and 4-formylmorpholine, a preferred availability may be ensured. *N,N*-Dimethylformamide is especially preferred. By using said *N,N*-dimethylformamide, an especially preferred availability may be ensured.

The *N,N*-dialkylformamide compound (9) may be used alone or in combination thereof, if necessary. The *N,N*-dialkylformamide compound (9) may be a commercially available one.

(b) A nucleophilic substitution reaction of the (6*Z*)-6-tridecenyl metal compound (1) with the *N,N*-dialkylformamide compound (9) will be explained in detail below.

The amount of the (6*Z*)-6-tridecenyl metal compound (1) used, per mol of the *N,N*-dialkylformamide compound (9), is preferably 0.5 to 1.5 mol. By using said preferred amount, preferred economy may be ensured.

Examples of the solvent used in the nucleophilic substitution reaction include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. Tetrahydrofuran, 2-methyltetrahydrofuran, 4-methyltetrahydropyran, and toluene are preferred. By using said tetrahydrofuran, 2-methyltetrahydrofuran, 4-methyltetrahydropyran, and toluene, a preferred reactivity may be ensured.

The solvent may be used alone or in combination thereof, if necessary. The solvent may be a commercially available one.

The amount of the solvent used, per mol of the *N,N*-dialkylformamide compound (9), is preferably 0 to 6,000 g. By using said preferred amount, a preferred reactivity may be ensured.

The reaction temperature of the nucleophilic substitution reaction is preferably - 20 to 40°C, more preferably -15 to 25°C, and especially preferably -10 to 15°C. By using said preferred reaction temperature, said more preferred reaction temperature, and said especially preferred reaction temperature, a preferred reactivity, a more preferred reactivity, and an especially preferred reactivity may be ensured.

The reaction time of the nucleophilic substitution reaction varies, depending on the solvent to be used or the production scale, and is preferably 0.5 to 100 hours. By using said preferred reaction time, a preferred reactivity may be ensured.

Next, a process for mixing the (6*Z*)-6-tridecenyl metal compound (1) and the *N,N*-dialkylformamide compound (9) in the nucleophilic substitution reaction will be explained in detail below. Initially, it was considered that the reactivity would not be affected by the process for mixing the (6*Z*)-6-tridecenyl metal compound (1) and the *N,N*-dialkylformamide compound (9) in the nucleophilic substitution reaction. However, contrary to expectations, adding the *N,N*-dialkylformamide compound (9) dropwise to the (6*Z*)-6-tridecenyl metal compound (1), as in Example 9 below, reduced the yield of the product, (7*Z*)-7-tetradecenal (8), by 5 to 10% compared to when the (6*Z*)-6-tridecenyl metal compound (1) was added dropwise to the *N,N*-dialkylformamide compound (9), as in Example 8 below. Although (7*Z*)-7-tetradecenal (8) may be obtained using either the process of adding the *N,N*-dialkylformamide compound (9) dropwise to the (6*Z*)-6-tridecenyl metal compound (1) or the process of adding the (6*Z*)-6-tridecenyl metal compound (1) dropwise to the *N,N*-dialkylformamide compound (9), the process of adding the (6*Z*)-6-tridecenyl metal compound (1) dropwise to the *N,N*-dialkylformamide compound (9) is preferred. By using said process of adding the (6*Z*)-6-tridecenyl metal compound (1) dropwise to the *N,N*-dialkylformamide compound (9), the aforesaid reduction of the yield of the product may be prevented. Without being bound by theory, the cause of the yield reduction when the *N,N*-dialkylformamide compound (9) is added dropwise to the (6*Z*)-6-tridecenyl metal compound (1) is considered to be oligomerization of reaction products due to the strong basicity of the (6*Z*)-6-tridecenyl metal compound (1) (a Grignard reagent).

Thus, the sex pheromone of the Olive moth, (7*Z*)-7-tetradecenal (8), may be efficiently prepared with fewer steps from the synthetic intermediate, (6*Z*)-6-tridecenyl metal compound (1).

## EXAMPLES

The present invention will be described with reference to the following Examples and Reference Examples. It should be noted that the present invention is not limited to or by the Examples.

The term "purity" as used herein means an area percentage in gas chromatography (GC), unless otherwise specified. The term "product ratio" means a ratio of area percentages in GC. The term "yield" is calculated from the area percentages determined by GC.

In the Examples, monitoring of the product ratios *E*/*Z* was carried out in the following GC conditions.

GC conditions: GC: Capillary gas chromatograph Agilent 7890B (Agilent Technologies Japan, Ltd.); column: SP-2331, 0.20 μm x 0.25 mmϕ x 60 m; carrier gas: He (1.00 mL/min), detector: FID; column temperature: 160°C.

In the Examples, monitoring of the reactions and calculation of the yields were carried out in the following GC

conditions.

GC conditions: GC: Capillary gas chromatograph GC-2014 (Shimadzu Corporation); column: DB-WAX, 0.25 μm x 0.25 mmϕ x 30 m; carrier gas: He (1.55 mL/min), detector: FID; column temperature: 150°C, elevated at a rate of 5°C/min up to 230°C.

The yield was calculated according to the following equation in consideration of purities (% GC) of a starting material and a product.

Yield (%) = {[(weight of a product obtained by a reaction × % GC) / molecular weight of a product] ÷ [(weight of a starting material in a reaction × % GC) / molecular weight of a starting material] × 100

THF represents tetrahydrofuran, DMF represents *N,N*-dimethylformamide, Et represents an ethyl group, and Ac represents an acetyl group.

Example 1: Preparation of 1-chloro-(6Z)-6-tridecene (5: $X^4$ = Cl)

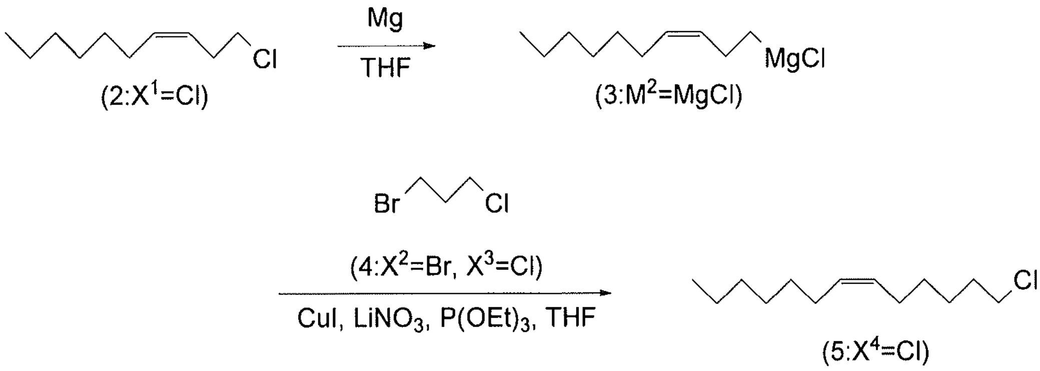


Magnesium (29.9 g, 1.23 gram atoms) and tetrahydrofuran (360.00 g) were placed in a reactor at a room temperature and stirred for 15 minutes at 60 to 65°C. 1-Chloro-(3Z)-3-decene (2: $X^1$ = Cl) (215.25 g, 1.200 mol, purity 97.40%, *E/Z*= 2.4/97.6) was then added dropwise at 60 to 75°C. After the completion of the dropwise addition, the reaction mixture was stirred for 2 hours at 75 to 80°C to obtain (3Z)-3-decenylmagnesium chloride (3: $M^2$ = MgCl).

Next, cuprous iodide (CuI) (2.50 g, 0.013 mol), lithium nitrate ($LiNO_3$) (0.93 g, 0.013 mol), triethyl phosphite ($P(OEt)_3$) (5.44 g, 0.033 mol), tetrahydrofuran (201.0 g), and 1-bromo-3-chloropropane (4: $X^2$ = Br, $X^3$ = Cl) (188.93 g, 1.200 mol) were placed in another reactor and cooled until the internal temperature of the other reactor became - 5°C, and then the aforesaid (3Z)-3-decenylmagnesium chloride (3: $M^2$ = MgCl) that was prepared was added dropwise. Although the internal temperature tends to rise due to heat of reaction as the dropwise addition proceeds, the drip rate and the cooling efficiency of the other reactor were controlled so that the internal temperature did not exceed 15°C (the greater part of the reaction temperature during the reaction was 10 to 15°C). After the completion of the dropwise addition, the reaction mixture was stirred for 2 hours at 10 to 25°C. An aqueous solution of ammonium chloride (prepared from ammonium chloride (13.00 g) and water (348.5 g)) and 20% by mass hydrochloric acid (15.13 g) were then added to the reaction mixture, and an aqueous solution (9.00 g) of 25% by mass sodium hydroxide was added to adjust the pH of the solution, followed by phase separation. The organic layer thus obtained was then concentrated at a reduced pressure, and the resulting concentrate was distilled at a reduced pressure to obtain 1-chloro-(6Z)-6-tridecene (5: $X^4$ = Cl) (195.64 g, 0.8918 mol, purity 98.82%, E/Z= 3.5/96.5, b.p. = 107.0 to 110.0°C/0.4 kPa, main distillate fraction yield 74.32%) with a total yield of 85.75%. The main distillate fraction yield refers to the yield of the main distillate fraction alone, and the total yield refers to the total yield of all distillate fractions.

The following is the spectrum data of 1-chloro-(6Z)-6-tridecene (5: $X^4$ = Cl) thus obtained.

Nuclear magnetic resonance spectrum: $^1$H-NMR (500 MHz, $CDCl_3$): δ = 0.89 (3H, t, J = 7.1 Hz), 1.23-1.48 (12H, m), 1.78 (2H, tt, J = 6.9 Hz, 6.9 Hz), 2.02 (2H, dt, J = 6.9 Hz, 6.7 Hz), 2.04 (2H, dt, J = 6.9 Hz, 6.7 Hz), 3.53 (2H, t, J = 6.9 Hz), 5.31-5.40 (2H, m); $^{13}$C-NMR (125 MHz, $CDCl_3$): δ = 14.09, 22.64, 26.52, 26.97, 27.23, 28.97, 29.69, 31.77, 32.55, 45.07, 129.24, 130.35.

Mass spectrum: EI-mass spectrum (70 eV): m/z 216 ($M^+$), 137, 123, 109, 97, 83, 69, 55, 41, 29.

Infrared absorption spectrum: (D-ATR): νmax = 3005, 2955, 2927, 2856, 1654, 1461, 1287, 728, 655.

Example 2: Preparation of 1-chloro-(6Z)-6-tridecene (5: $X^4$ = Cl)

A reaction was carried out as in Example 1, with the proviso that the reaction temperature of the coupling reaction was modified to 35 to 40°C, thereby forming 1-chloro-(6Z)-6-tridecene (5: $X^4$ = Cl) (181.88 g, 0.8286 mol, purity 98.76%, E/Z= 6.1/94.0, b.p. = 107.0 to 110.0°C/0.4 kPa, main distillate fraction yield 69.05%) with a total yield of 76.64%.
The spectrum data of 1-chloro-(6Z)-6-tridecene (5: $X^4$ = Cl) thus obtained was the same as that of Example 1.

Example 3: Preparation of (6*Z*)-6-tridecenylmagnesium chloride (1: $M^1$ = MgCl)

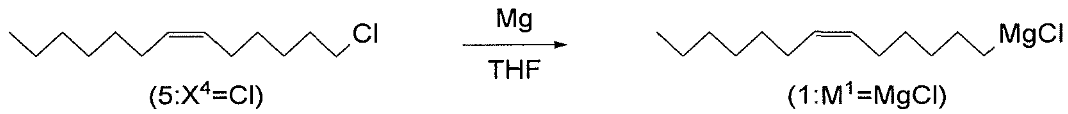


(5:$X^4$=Cl) (1:$M^1$=MgCl)

Magnesium (14.87 g, 0.612 gram atoms) and tetrahydrofuran (180.0 g) were placed in a reactor at a room temperature and stirred for 20 minutes at 60 to 65°C. 1-Chloro-(6*Z*)-6-tridecene (5: $X^4$ = Cl) (131.84 g, 0.6000 mol, purity 98.66%, *E*/*Z* = 3.5/96.5) prepared as in Example 1 was then added dropwise at 65 to 75°C. After the completion of the dropwise addition, the reaction mixture was stirred for 2 hours at 75 to 80°C to obtain (6*Z*)-6-tridecenylmagnesium chloride (1: $M^1$ = MgCl).
The following is the spectrum data of a THF solution of (6*Z*)-6-tridecenylmagnesium chloride (1: $M^1$ = MgCl) thus obtained.
Nuclear magnetic resonance spectrum: $^1$H-NMR (600 MHz, THF-$d_8$): δ = -0.71 (2H, t, J = 7.8 Hz), 0.83 (3H, t, J = 6.9 Hz), 1.15-1.32 (12H, m), 1.47 (2H, tt, J = 7.8 Hz, 7.2 Hz), 1.90-2.02 (4H, m), 5.18-5.23 (1H, m), 5.25-5.31 (1H, m); $^{13}$C-NMR (150 MHz, THF-$d_8$): δ = 7.82, 14.14, 23.20, 27.67, 28.21, 29.56, 30.40, 30.61, 30.78, 32.41, 39.22, 129.24, 131.22.
Infrared absorption spectrum: (D-ATR): νmax = 2957, 2924, 2854, 1651, 1460, 1180, 1069, 1035, 914, 882, 725, 678, 551, 508.

Example 4: Preparation of 1-bromo-(6Z)-6-tridecene (5: $X^4$ = Br)

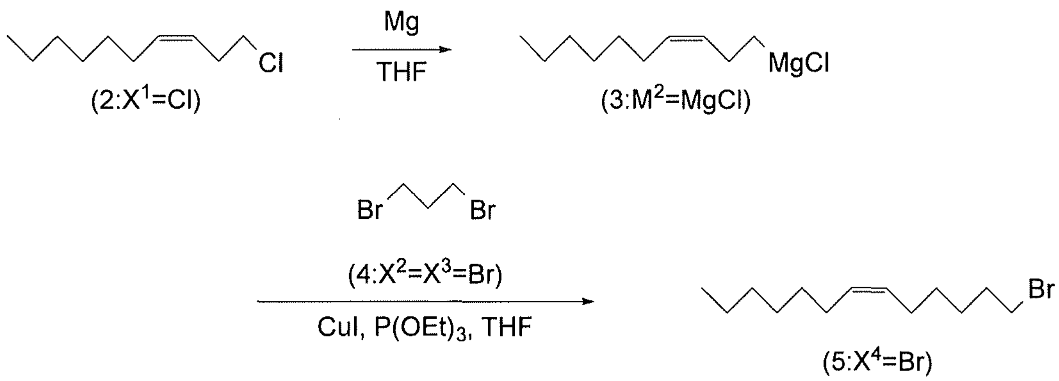


(2:$X^1$=Cl) (3:$M^2$=MgCl) (4:$X^2$=$X^3$=Br) (5:$X^4$=Br)

Magnesium (7.47 g, 0.307 gram atoms) and tetrahydrofuran (90.00 g) were placed in a reactor at a room temperature and stirred for 15 minutes at 60 to 65°C. 1-Chloro-(3*Z*)-3-decene (2: $X^1$ = Cl) (53.81 g, 0.3000 mol, purity 97.40%, *E*/*Z* = 2.4/97.6) was then added dropwise at 60 to 75°C. After the completion of the dropwise addition, the reaction mixture was stirred for 2 hours at 75 to 80°C to obtain (3Z)-3-decenylmagnesium chloride (3: $M^2$ = MgCl).
Next, cuprous iodide (CuI) (3.06 g, 0.016 mol), triethyl phosphite ($P(OEt)_3$) (3.06 g, 0.018 mol), tetrahydrofuran (88.0 g), and 1,3-dibromopropane (4: $X^2$ = $X^3$ = Br) (122.95 g, 0.6090 mol) were placed in another reactor, and then the aforesaid (3Z)-3-decenylmagnesium chloride (3: $M^2$ = MgCl) that was prepared was added dropwise at -5 to 10°C. After the completion of the dropwise addition, the reaction mixture was stirred for 2 hours at 10 to 25°C. An aqueous solution of ammonium chloride (prepared from ammonium chloride (6.70 g) and water (139.0 g)) and 20% by mass hydrochloric acid (2.2 g) were then added to the reaction mixture, followed by phase separation. The organic layer thus obtained was concentrated at a reduced pressure, and the resulting concentrate was distilled at a reduced pressure to obtain 1-bromo-(6*Z*)-6-tridecene (5: $X^4$ = Br) (45.28 g, 0.1705 mol, purity 98.36%, *E*/*Z*= 4.4/95.6, b.p. = 81.0 to 87.0°C/0.02 kPa,

main distillate fraction yield 56.83%) with a total yield of 77.93%.

The following is the spectrum data of 1-bromo-(6*Z*)-6-tridecene (5: $X^4$ = Br) thus obtained.

Nuclear magnetic resonance spectrum: $^1$H-NMR (500 MHz, $CDCl_3$): δ = 0.88 (3H, t, J = 7.1 Hz), 1.22-1.48 (12H, m), 1.86 (2H, tt, J = 7.3 Hz, 6.9 Hz), 2.01 (2H, dt, J = 6.9 Hz, 6.7 Hz), 2.04 (2H, dt, J = 6.9 Hz, 6.8 Hz), 3.40 (2H, t, J = 6.9 Hz), 5.30-5.40 (2H, m); $^{13}$C-NMR (125 MHz, $CDCl_3$): δ = 14.09, 22.64, 26.95, 27.23, 27.81, 28.85, 28.97, 29.68, 31.76, 32.73, 33.85, 129.21, 130.38.

Mass spectrum: EI-mass spectrum (70 eV): m/z 260 ($M^+$), 190, 176, 162, 148, 125, 111, 97, 83, 69, 55, 41, 27.

Infrared absorption spectrum: (D-ATR): νmax = 3005, 2956, 2926, 2855, 1460, 1378, 1268, 727, 647, 564.

Example 5: Preparation of (6*Z*)-6-tridecenylmagnesium bromide (1: $M^1$ = MgBr)

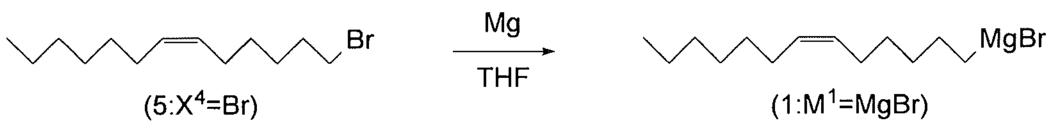


(5:$X^4$=Br) (1:$M^1$=MgBr)

Magnesium (1.86 g, 0.0765 gram atoms) and tetrahydrofuran (22.50 g) were placed in a reactor at a room temperature and stirred for 20 minutes at 60 to 65°C. 1-Bromo-(6*Z*)-6-tridecene (5: $X^4$ = Br) (19.98 g, 0.0751 mol, purity 98.36%, *E*/*Z*= 4.4/95.6) prepared in Example 4 was then added dropwise at 60 to 70°C. After the completion of the dropwise addition, the reaction mixture was stirred for 2 hours at 60 to 70°C to form (6*Z*)-6-tridecenylmagnesium bromide (1: $M^1$ = MgBr).

The following is the spectrum data of a THF solution of (6*Z*)-6-tridecenylmagnesium bromide (1: $M^1$ = MgBr) thus obtained.

Nuclear magnetic resonance spectrum: $^1$H-NMR (600 MHz, THF-$d_8$): δ = -0.71 (2H, t, J = 7.8 Hz), 0.82 (3H, t, J = 7.1 Hz), 1.15-1.33 (12H, m), 1.46 (2H, tt, J = 7.8 Hz, 7.2 Hz), 1.89-2.01 (4H, m), 5.17-5.23 (1H, m), 5.24-5.35 (1H, m); $^{13}$C-NMR (150 MHz, THF-$d_8$): δ = 7.93, 14.12, 23.14, 27.62, 28.13, 29.50, 30.33, 30.53, 30.67, 32.34, 39.10, 129.21, 131.12.

Infrared absorption spectrum: (D-ATR): νmax = 2956, 2924, 2854, 1651, 1459, 1179, 1069, 1032, 914, 880, 725, 679, 551, 507.

Example 6 Preparation of 1,1-diethoxy-(7*Z*)-7-tetradecene (7: $R^4$ = $R^5$ = Et)

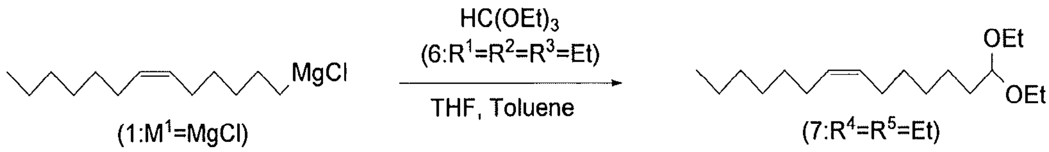


(1:$M^1$=MgCl) (7:$R^4$=$R^5$=Et)

A tetrahydrofuran solution (322.82 g) of (6*Z*)-6-tridecenylmagnesium chloride (1: $M^1$ = MgCl) (0.6000 mol) prepared as in Example 3 and toluene (300.0 g) were placed in a reactor at a room temperature and heated to 85 to 90°C. Ethyl orthoformate ($HC(OEt)_3$) (6: $R^1$ = $R^2$ = $R^3$ = Et) (115.38 g, 0.786 mol) was then added dropwise at 85 to 95°C. After the completion of the dropwise addition, the reaction mixture was stirred for 13 hours at 85 to 95°C, and then the reaction mixture was cooled to 20 to 35°C. Acetic acid (44.5 g) and water (289.3 g) were added to the reaction mixture, followed by phase separation. The aqueous layer was removed, and the organic layer was then concentrated at a reduced pressure. The resulting concentrate was distilled at a reduced pressure to obtain 1,1-diethoxy-(7Z)-7-tetradecene (7: $R^4$ = $R^5$ = Et) (125.61 g, 0.4368 mol, purity 98.93%, *E*/*Z* = 3.5/96.5, b.p. = 111.0 to 128.0°C/0.2 kPa, main distillate fraction yield 72.80%) with a total yield of 80.08%.

The following is the spectrum data of 1,1-diethoxy-(7Z)-7-tetradecene (7: $R^4$ = $R^5$ = Et) thus obtained.

Nuclear magnetic resonance spectrum: $^1$H-NMR (500 MHz, $CDCl_3$): 0.87 (3H, t, J = 7.1 Hz), 1.19 (6H, t, J = 7.1 Hz), 1.22-1.39 (14H, m), 1.57-1.62 (2H, m), 1.98-2.04 (4H, m), 3.47 (1H, q, J = 7.1 Hz), 3.49 (1H, q, J = 7.0 Hz), 3.62 (1H, q, J = 7.1 Hz), 3.64 (1H, q, J = 7.0 Hz), 4.47 (1H, t, J = 5.7 Hz), 5.30-5.39 (2H, m); $^{13}$C-NMR (125 MHz, $CDCl_3$): δ = 14.08, 15.33, 22.63, 24.63, 27.10, 27.20, 28.97, 29.11, 29.65, 29.71, 31.76, 33.53, 60.77, 102.90, 129.68, 129.99.

Mass spectrum: EI-mass spectrum (70 eV): m/z 239 ($M^+$-45), 223, 192, 156, 142, 121, 103, 75, 47, 29.

Infrared absorption spectrum: (D-ATR): νmax = 3004, 2974, 2927, 2856, 1459, 1375, 1344, 1128, 1063, 1001, 725.

Example 7: Preparation of (7*Z*)-7-tetradecenal (8)

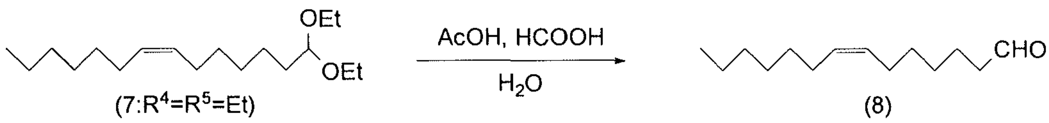


(7:$R^4$=$R^5$=Et)

(8)

1,1-Diethoxy-(7*Z*)-7-tetradecene (7: $R^4$ = $R^3$ = Et) (125.61 g, 0.4368 mol, purity 98.93%, *E*/*Z*= 3.5/96.5) prepared in Example 6, acetic acid (AcOH) (45.25 g, 0.754 mol), formic acid (HCOOH) (18.40 g, 0.400 mol), and water ($H_2O$) (32.22 g, 1.79 mol) were placed in a reactor at a room temperature and stirred for 2.5 hours at 90 to 100°C. The reaction mixture was then cooled to 40 to 55°C and left to stand for phase separation, followed by removal of the aqueous layer to obtain the organic layer. The organic layer thus obtained was then concentrated at a reduced pressure, and the resulting concentrate was distilled at a reduced pressure to obtain (7Z)-7-tetradecenal (8) (87.48 g, 0.4091 mol, purity 98.37%, *E*/*Z* = 3.8/96.2, b.p. = 95.0 to 110.0°C/0.2 kPa, main distillate fraction yield 93.65%) with a total yield of 93.65%.

The following is the spectrum data of (7*Z*)-7-tetradecenal (8) thus obtained.

Nuclear magnetic resonance spectrum: $^1$H-NMR (500 MHz, $CDCl_3$): δ = 0.87 (3H, t, J = 7.1 Hz), 1.21-1.40 (12H, m), 1.63 (2H, tt, J = 7.3 Hz, 7.3 Hz), 2.00 (2H, dt, J = 6.9 Hz, 6.5 Hz), 2.02 (2H, dt, J = 6.5 Hz, 6.9 Hz), 2.42 (2H, td, J = 7.3 Hz, 1.7 Hz), 5.28-5.39 (2H, m), 9.76 (1H, t, J = 1.7 Hz); $^{13}$C-NMR (125 MHz, $CDCl_3$): δ = 14.07, 21.95, 22.62, 26.91, 27.20, 28.75, 28.95, 29.41, 29.67, 31.74, 43.85, 129.30, 130.26, 202.77.

Mass spectrum: EI-mass spectrum (70 eV): m/z 192 ($M^+$-18), 163, 149, 135, 121, 98, 81, 67, 55, 41, 29.

Infrared absorption spectrum: (D-ATR): νmax = 3005, 2927, 2856, 2714, 1728, 1462, 1409, 725.

Example 8: Preparation of (7*Z*)-7-tetradecenal (8)

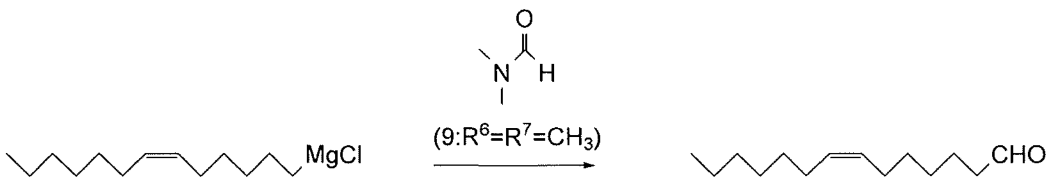


(1:$M^1$=MgCl)

THF

(8)

*N,N*-Dimethylformamide (9: $R^6$ = $R^7$ = $CH_3$) (12.06 g, 0.1650 mol) and tetrahydrofuran (30.00 g) were placed in a reactor at a room temperature. A tetrahydrofuran solution (81.68 g) of (6*Z*)-6-tridecenylmagnesium chloride (1: $M^1$ = MgCl) (0.1500 mol) prepared as in Example 3 was then continuously added dropwise with a dropping funnel for 1.5 hours at 0 to 5°C. After the completion of the dropwise addition, the reaction mixture was stirred for 1 hour at 0 to 5°C.

Next, acetic acid (20.72 g, 0.345 mol) and water (82.88 g, 6.90 mol) were placed in another container, and the aforesaid reaction mixture was added dropwise at 0 to 20°C. After the completion of the dropwise addition, the reaction mixture was stirred for 1.5 hours at 10 to 20°C. After the completion of the stirring, the reaction mixture was left to stand for phase separation, followed by removal of the aqueous layer to obtain the organic layer. The organic layer thus obtained was then concentrated at a reduced pressure, and the resulting concentrate was distilled at a reduced pressure to obtain (7*Z*)-7-tetradecenal (8) (22.96 g, 0.1051 mol, purity 96.32%, *E*/*Z* = 3.7/96.3, b.p. = 93.0 to 99.0°C/0.1 kPa, main distillate fraction yield 70.06%) with a total yield of 85.40%.

The spectrum data of (7*Z*)-7-tetradecenal (8) thus obtained was the same as that obtained in Example 7.

Example 9: Preparation of (7*Z*)-7-tetradecenal (8)

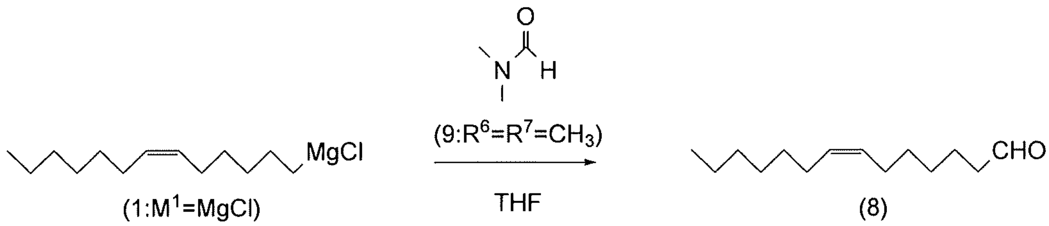


A tetrahydrofuran solution (81.68 g) of (6*Z*)-6-tridecenylmagnesium chloride (1: $M^1$ = MgCl) (0.1500 mol) prepared as in Example 3 was placed in a reactor at a room temperature. A mixture of *N,N*-dimethylformamide (9: $R^6$ = $R^7$ = $CH_3$) (12.06 g, 0.1650 mol) and tetrahydrofuran (30.00 g) was continuously added dropwise with a dropping funnel for 1.5 hours at 0 to 5°C. After the completion of the dropwise addition, the reaction mixture was stirred for 1 hour at 0 to 5°C.

Next, acetic acid (20.72 g, 0.345 mol) and water (82.88 g, 6.90 mol) were placed in another container, and the aforesaid reaction mixture was added dropwise at 0 to 20°C. After the completion of the dropwise addition, the reaction mixture was stirred for 1.5 hours at 10 to 20°C. After the completion of the stirring, the reaction mixture was left to stand for phase separation, followed by removal of the aqueous layer to obtain the organic layer. The organic layer thus obtained was then concentrated at a reduced pressure, and the resulting concentrate was distilled at a reduced pressure to obtain (7*Z*)-7-tetradecenal (8) (21.67 g, 0.09801 mol, purity 95.14%, *E*/*Z* = 3.7/96.3, b.p. = 93.0 to 99.0°C/0.1 kPa, main distillate fraction yield 65.34%) with a total yield of 78.73%.

The spectrum data of (7*Z*)-7-tetradecenal (8) thus obtained was the same as that obtained in Example 7.

Reference Example 1: Preparation of 1-chloro-(10*Z*)-10-tridecene

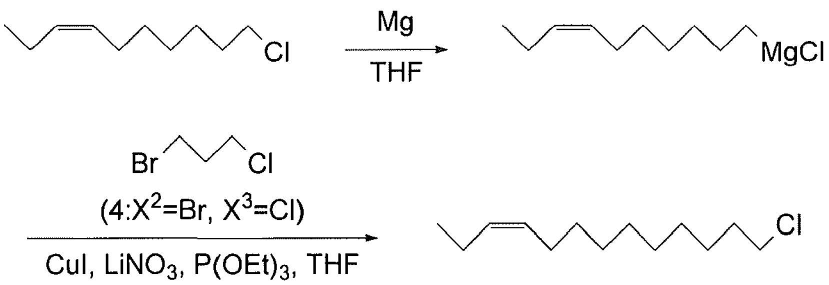


Magnesium (29.9 g, 1.23 gram atoms) and tetrahydrofuran (360.00 g) were placed in a reactor at a room temperature and stirred for 15 minutes at 60 to 65°C. 1-Chloro-(7*Z*)-7-decene (213.58 g, 1.200 mol, purity 98.16%, *E*/*Z* = 3.3/96.7) was then added dropwise at 60 to 75°C. After the completion of the dropwise addition, the reaction mixture was stirred for 2 hours at 75 to 80°C to form (7*Z*)-7-decenylmagnesium chloride.

Next, cuprous iodide (CuI) (2.50 g, 0.013 mol), lithium nitrate ($LiNO_3$) (0.93 g, 0.013 mol), triethyl phosphite ($P(OEt)_3$) (5.44 g, 0.033 mol), tetrahydrofuran (201.0 g), and 1-bromo-3-chloropropane (4: $X^2$ = Br, $X^3$ = Cl) (188.93 g, 1.200 mol) were placed in another reactor. The aforesaid (7*Z*)-7-decenylmagnesium chloride that was prepared was then added dropwise at 35 to 40°C. After the completion of the dropwise addition, the reaction mixture was stirred for 2 hours at 35 to 40°C. An aqueous solution of ammonium chloride (prepared from ammonium chloride (13.00 g) and water (348.5 g)) and 20% by mass hydrochloric acid (23.83 g) were then added to the reaction mixture. An aqueous solution (12.00 g) of 25% by mass sodium hydroxide was then added to adjust the pH of the solution, followed by phase separation. The organic layer thus obtained was then concentrated at a reduced pressure, and the resulting concentrate was distilled at a reduced pressure to obtain 1-chloro-(10*Z*)-10-tridecene (189.07 g, 0.8498 mol, purity 97.44%, *E*/*Z* = 3.3/96.7, b.p. = 103.0 to 107.0°C/0.3 kPa, main distillate fraction yield 70.82%) with a total yield of 73.13%.

The following is the spectrum data of 1-chloro-(10*Z*)-10-tridecene thus obtained.

Nuclear magnetic resonance spectrum: $^1$H-NMR (500 MHz, $CDCl_3$): $\delta$ = 0.96 (3H, t, J = 7.6 Hz), 1.25-1.46 (12H, m), 1.76 (2H, tt, J = 7.2 Hz, 6.9 Hz), 2.02 (2H, dt, J = 6.9 Hz, 6.5 Hz), 2.04 (2H, dt, J = 7.6 Hz, 6.9 Hz), 3.53 (2H, t, J = 6.9 Hz), 5.29-5.40 (2H, m); $^{13}$C-NMR (125 MHz, $CDCl_3$): $\delta$ = 14.37, 20.50, 26.88, 27.06, 28.87, 29.21, 29.41, 29.73, 32.64, 45.14, 129.26, 131.54.

Mass spectrum: EI-mass spectrum (70 eV): m/z 216 ($M^+$), 160, 137, 123, 111, 97, 83, 69, 55, 41, 29.

Infrared absorption spectrum: (D-ATR): νmax = 3004, 2961, 2927, 2855, 1463, 1305, 1069, 724, 655.

## Claims

1. A (6Z)-6-tridecenyl metal compound of the following general formula (1):

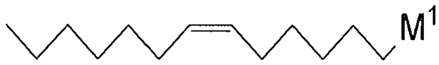 (1)

wherein $M^1$ represents Li or $MgZ^1$, and $Z^1$ represents a halogen atom or a (6*Z*)-6-tridecenyl group.

2. A process for preparing a (6*Z*)-6-tridecenyl metal compound of the following general formula (1):

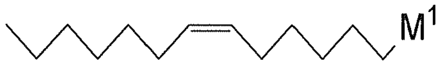 (1)

wherein $M^1$ represents Li or $MgZ^1$, and $Z^1$ represents a halogen atom or a (6*Z*)-6-tridecenyl group,
the process comprising steps of:
preparing a (3Z)-3-decenyl metal compound of the following general formula (3):

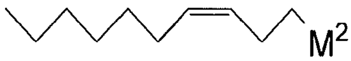 (3)

wherein $M^2$ represents Li or $MgZ^2$, and $Z^2$ represents a halogen atom or a (3*Z*)-3-decenyl group,
from a 1-halo-(3*Z*)-3-decene compound of the following general formula (2):

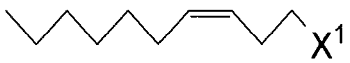 (2)

wherein $X^1$ represents a halogen atom;
subjecting the (3*Z*)-3-decenyl metal compound (3) to a coupling reaction with a 1,3-dihalopropane compound of the following general formula (4):

$X^2(CH_2)_3X^3$ (4)

wherein $X^2$ and $X^3$ represent, independently of each other, a halogen atom,
to form a 1-halo-(6*Z*)-6-tridecene compound of the following general formula (5):

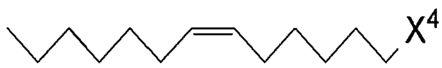 (5)

wherein $X^4$ represents a halogen atom; and
preparing the aforesaid (6Z)-6-tridecenyl metal compound (1) from the 1-halo-(6Z)-6-tridecene compound (5).

3. A process for preparing (7Z)-7-tetradecenal of the following formula (8):

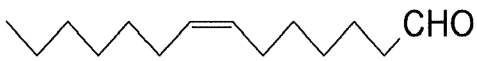 (8)

the process comprising steps of:
subjecting a (6*Z*)-6-tridecenyl metal compound of the following general formula (1):

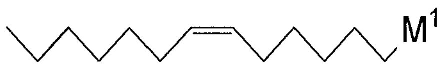 (1)

wherein $M^1$ represents Li or $MgZ^1$, and $Z^1$ represents a halogen atom or a (6*Z*)-6-tridecenyl group,
to a nucleophilic substitution reaction with an orthoformate ester compound of the following general formula (6):

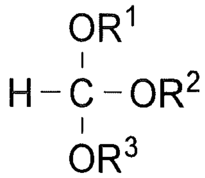

(6)

wherein $R^1$, $R^2$, and $R^3$ represent, independently of each other, an alkyl group having 1 to 6 carbon atoms, to form a 1,1-dialkoxy-(7*Z*)-7-tetradecene compound of the following general formula (7):

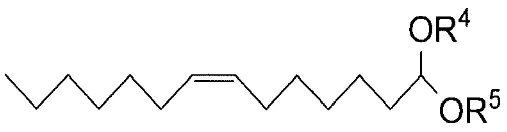

(7)

wherein $R^4$ and $R^5$ represent, independently of each other, an alkyl group having 1 to 6 carbon atoms; and subjecting the 1,1-dialkoxy-(7*Z*)-7-tetradecene compound (7) to a hydrolysis reaction to form the aforesaid (7*Z*)-7-tetradecenal (8).

**4.** A process for preparing (7*Z*)-7-tetradecenal of the following formula (8):

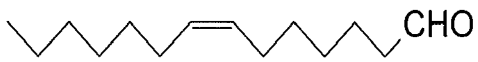

(8)

the process comprising a step of:
subjecting a (6*Z*)-6-tridecenyl metal compound of the following general formula (1):

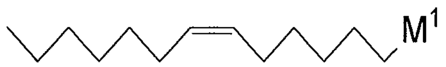

(1)

wherein $M^1$ represents Li or $MgZ^1$, and $Z^1$ represents a halogen atom or a (6*Z*)-6-tridecenyl group,
to a nucleophilic substitution reaction with an *N,N*-dialkylformamide compound of the following general formula (9):

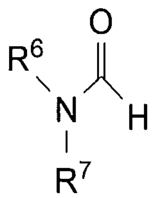

(9)

wherein $R^6$ and $R^7$ represent, independently of each other, an alkyl group having 1 to 3 carbon atoms, or $R^6$ and $R^7$ may be bonded to each other to form a divalent alkylene group having 2 to 6 carbon atoms or a 3-oxa-1,5-pentanediyl group,
to form the aforesaid (7*Z*)-7-tetradecenal (8).

**5.** The process for preparing (7*Z*)-7-tetradecenal (8) according to claim 4, wherein the (6*Z*)-6-tridecenyl metal compound (1) is added dropwise to the *N,N*-dialkylformamide compound (9) in the aforesaid nucleophilic substitution reaction.

Europäisches Patentamt
European Patent Office
Office européen des brevets

# EUROPEAN SEARCH REPORT

Application Number
EP 25 20 8247

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2023/012151 A1 (BIOPHERO APS [DK]) 9 February 2023 (2023-02-09) * page 95, line 1 * * claims 1,20 * * abstract * -& DATABASE CAPLUS [Online] 1 January 2023 (2023-01-01), Biophero Aps: "Large scale process preparation of fatty aldehydes from fatty alcohols - WO 2023012151 A1", XP093352205, Database accession no. 2023:279545 * abstract * ----- | 1-5 | INV. C07C41/48 C07C45/51 C07F3/02 |
| A | BECKER D. ET AL: "An alternative synthesis of unsaturated aldehydopheromones", TETRAHEDRON, vol. 38, no. 11, 1 January 1982 (1982-01-01), pages 1689-1692, XP093350994, AMSTERDAM, NL ISSN: 0040-4020, DOI: 10.1016/0040-4020(82)80148-8 * scheme *; page 1689 * page 1692, column 2, paragraph 1 * ----- | 1-5 | TECHNICAL FIELDS SEARCHED (IPC): C07C C07F |
| A | US 2018/305284 A1 (MIYAKE YUKI [JP] ET AL) 25 October 2018 (2018-10-25) * paragraph [0085] * ----- | 3-5 | |

1

The present search report has been drawn up for all claims

| Place of search | Date of completion of the search | Examiner |
|---|---|---|
| The Hague | 13 January 2026 | Eberhard, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 20 8247

This annex lists the patent family members relating to the patent documents cited in the above-mentioned European search report. The members are as contained in the European Patent Office EDP file on
The European Patent Office is in no way liable for these particulars which are merely given for the purpose of information.

13-01-2026

| Patent document cited in search report | Publication date | Patent family member(s) | Publication date |
|---|---|---|---|
| WO 2023012151 A1 | 09-02-2023 | AR 126717 A1 | 08-11-2023 |
| | | AU 2022321753 A1 | 25-01-2024 |
| | | CA 3225388 A1 | 09-02-2023 |
| | | CL 2024000256 A1 | 07-06-2024 |
| | | EP 4380916 A1 | 12-06-2024 |
| | | IL 309880 A | 01-03-2024 |
| | | JP 2024537933 A | 17-10-2024 |
| | | KR 20240042428 A | 02-04-2024 |
| | | TW 202313546 A | 01-04-2023 |
| | | US 2024327324 A1 | 03-10-2024 |
| | | WO 2023012151 A1 | 09-02-2023 |
| US 2018305284 A1 | 25-10-2018 | CN 108727170 A | 02-11-2018 |
| | | EP 3392231 A1 | 24-10-2018 |
| | | JP 6670793 B2 | 25-03-2020 |
| | | JP 2018177743 A | 15-11-2018 |
| | | US 2018305284 A1 | 25-10-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **E. M. HEGAZI et al.** *Crop Protection*, 2009, vol. 28, 181-189
- **D. G. CAMPION et al.** *Experientia*, 1979, vol. 35, 1146-1147
- **MASAO SHIOZAKI et al.** *Bull. Chem. Soc. Jpn.*, 2003, vol. 76, 1011-1022